# EUROPEAN PATENT APPLICATION

(11) **EP 2 778 153 A1**
(43) Date of publication of application: **17.09.2014**
(21) Application number: 12847393.1
(22) Date of filing: 06.11.2012
(51) Int. Cl.: C07C 67/05, C07C 69/15

(54) **METHOD FOR PRODUCING VINYL ACETATE**

(30) Priority: 11.11.2011 CN 201110357422
(71) Applicant: Tianjin University, Tianjin 300072 (CN)
(72) Inventor: The designation of the inventor has not yet been filed
(74) Representative: Walkenhorst, Andreas
(86) International application number: PCT/CN2012/084167
(87) International publication number: WO 2013/067920

(57) **Abstract**

The present invention discloses a method for producing vinyl acetate. Inert gas is introduced into cycling gas to improve the explosion limit of oxygen, enlarge a stable area of a reaction system and improve the oxygen concentration of the reaction system, thus reducing the reaction temperature, prolonging the service life of a catalyst, and improving the selectivity of the vinyl acetate. The inert gas is nitrogen or a mixture of nitrogen and methane. The concentration of the inert gas at the inlet of a reactor is 8 to 30 mol%. The molar concentration of the oxygen at the inlet of the reactor is 10.5 to 12 mol%.

## Description

### TECHNICAL FIELD

The present invention relates to a method for producing vinyl acetate. According to the present method, inert gas is introduced into cycling gas to expand a stable area and improve the concentration of oxygen at the inlet of a reactor, thus reducing the reaction temperature, prolonging the service life of a catalyst, improving the selectivity of the vinyl acetate, reducing the production cost, and enhancing the market competitiveness of vinyl acetate products.

### BACKGROUND ART

Vinyl acetate (VAC) is an important organic chemical raw material which can generate such derivatives as polyvinyl alcohol (PVA), ethylene vinyl acetate copolymer (EVA), polyvinyl acetate (PVAC), and vinyl acetate - vinyl chloride copolymer (EVC) through self-polymerization or copolymerization with other monomer. These derivatives are widely used in many aspects, such as adhesive, adhesive agents in paper or fabric, paint, ink, leather processing, emulsifier, water soluble film, soil-improvement agent and so on. And there is a growing need of VAC as intermediates along with the extensive non-fiber applications field of polyvinyl alcohol.

The production of polyvinyl alcohol in our country began from 1960s. An "acetylene fluidized bed technology" technology was introduced from Japan by Beijing Organic Chemical Plant in 1965, and multiple suits of similar devices were established at home later. These vinyl acetate devices were matched with vinylon, and about 90 % of vinyl acetate in China was used for vinylon and polyvinyl alcohol in the 1970s to 1980s. Along with the development of polyvinyl alcohol, the market of vinyl acetate in China has been expanded continuously in recent years. By the end of the 2008, the total productive capacity of vinyl acetate in China reached 1.4 million tons per year, and the demand for VAC is expected to grow at a rate of 8 % in China in the future.

There are two methods of ethylene method and acetylene method in vinyl acetate production process route. The acetylene method has been abandoned in foreign countries because of serious pollution, and is adopted in only a few countries such as China adopt at present. The production with ethylene method is dominated around the world. During the production process of vinyl acetate with ethylene method, raw materials of ethylene, oxygen and acetic acid gas are introduced in a reactor and contacted with a catalyst in the reactor so as to produce VAC, water and a small amount of byproducts under the absolution pressure of 0.6-1.1 MPa and at the temperature of 130-200 °C through reaction. Through multi-stage condensation of high-temperature reaction gas, vinyl acetate, water, unreacted acetic acid and the like are contained in a condensed fluid, and mixed solution is sent to a rectification working procedure for refining of the VAC. The unreacted acetic acid gas is returned to a compressor for recycling, and is called cycling gas.

During the production process of vinyl acetate through ethylene gas phase oxidation, the improvement of vinyl acetate yield is related to the concentration of oxygen in the system. By improving the concentration of the oxygen properly, the reaction temperature can be reduced, the service life of the catalyst is prolonged, and the selectivity of the vinyl acetate is improved, thus reducing the production cost, improving the market competitiveness of vinyl acetate products. However, the improvement of the concentration of the oxygen in the system is limited by explosion limit, and the concentration of the oxygen is usually controlled in a lower range (smaller than or equal to 6 % mol%) in actual production. The lower concentration of the oxygen has higher operational safety, but the reaction temperature is higher, the single-way conversion rate of ethylene is lower, and the lower concentration of the oxygen is economically disadvantageous. Therefore, during the production process of synthesizing vinyl acetate and under the precondition of meeting the safety, a new technological process is required to improve the concentration of the oxygen, so that the reaction temperature can be reduced, and the selectivity of the vinyl acetate is improved, thus the production cost can be reduced.

### SUMMARY OF THE INVENTION

The present invention aims to provide a method for producing vinyl acetate. Said method can improve the concentration of oxygen at the inlet of a reactor, thus reducing the reaction temperature, prolonging the service life of a catalyst, improving the selectivity of the vinyl acetate, reducing the production cost, and enhancing the yield of the vinyl acetate.

During the production process of vinyl acetate with ethylene method, raw materials of ethylene, oxygen and acetic acid gas are introduced in the reactor and contacted with the catalyst in the reactor so as to produce the vinyl acetate, water and a small amount of byproducts through reaction. At the initial period of using the catalyst, the reaction temperature is 130 °C; at the middle period of using the catalyst, the reaction temperature is 170 °C; and at the later period of using the catalyst, the reaction temperature is finally up to 200 °C along with the decrease of the activity of the catalyst. Through multi-stage condensation of high-temperature reaction gas, vinyl acetate, water, unreacted acetic acid and the like are contained in a condensed fluid, and mixed solution is sent to a rectification working procedure for refining of the vinyl acetate. Unreacted ethylene, oxygen and the like contained in uncondensed gas are returned to a compressor for recycling, and are called cycling gas.

During the production process of vinyl acetate with vapor phase method, the improvement of vinyl acetate yield is related to the concentration of the oxygen in the system. In a certain concentration range, improving the concentration of the oxygen in the reactor can reduce the reaction temperature, prolong the service life of the catalyst, and improve the reaction selectivity. However, the improvement of the concentration of the oxygen in the system is limited by explosion limit, and the concentration of the oxygen is usually controlled in a lower range in actual production so as to cause the increase of the reaction temperature and the decrease of the selectivity of the vinyl acetate. The explosion limit of the oxygen is a function consisting of temperature, pressure and mixture, thus the explosion limit of the oxygen can be changed by changing the composition of temperature, pressure and mixture. For example, the explosion lower limit of the oxygen can be improved by improving the content of acetic acid in the system so as to play a role of enlarging a stable area. However, acetic acid steam in the system is obtained by heating fresh steam, and the adoption of the method may cause the energy consumption of the system and the increase of the production cost.

The present invention discloses a method for producing vinyl acetate. Inert gas is added into cycling gas to improve the explosion lower limit of the oxygen, enlarge a stable area of a reaction system and improve the oxygen concentration of the reaction system, thus reducing the reaction temperature, prolonging the service life of the catalyst, and improving the selectivity of the vinyl acetate.

According to the method for producing vinyl acetate disclosed by the present invention, the inert gas is introduced into the cycling gas, and the concentration of the inert gas at the inlet of the reactor is 8 to 30 mol%.

The inert gas is nitrogen or a mixture of nitrogen and methane.

When the inert gas is the mixture of nitrogen and methane, the volume ratio of nitrogen to methane is 4:1 to 4:2.

Through the adoption of the above means, the molar concentration of the oxygen at the inlet of the reactor during the actual production process can reach to 10.5-12 mol%.

According to the method, the reaction temperature is 110-180 °C and is reduced by 5-20 °C, and the selectivity of the vinyl acetate is 93.5-96 % and improved by 0.5-3 %.

The method for producing vinyl acetate disclosed by the present invention has the advantage that the concentration of the inert gas in the system is improved so as to improve the explosion limit of the oxygen and enlarge the stable area of the reaction system, thus improving the actual concentration of the oxygen at the inlet of the reactor during the production process. The stable area is enlarged without improving the content of the acetic acid in the system, thus the content of the acetic acid in the cycling gas is reduced, the steam consumption is reduced, the production cost is reduced, and the market competitiveness of vinyl acetate products is improved.

### BRIEF DESCRIPTION OF DRAWINGS

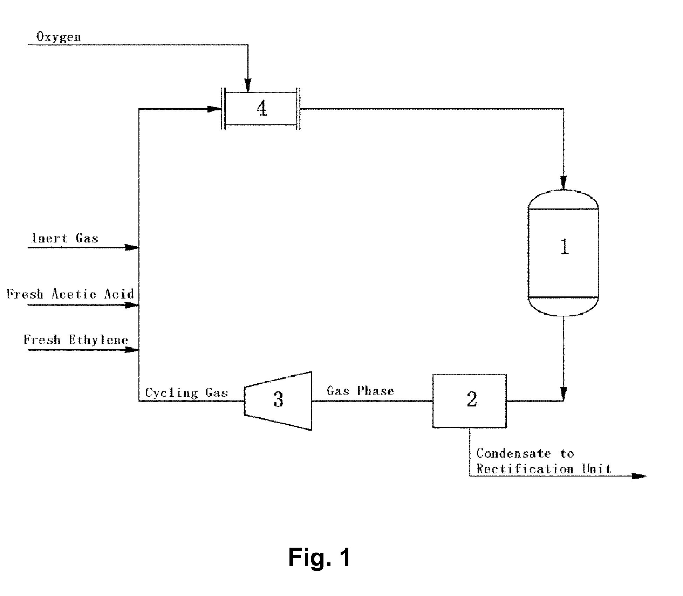

Fig. 1: a flow diagram of production process of vinyl acetate.

### DETAILED DESCRIPTION OF THE INVENTION

The following further describes implementation process of the present invention in detail with reference to specific embodiments. The reaction pressure is absolute pressure of 0.6-1.1 MPa.

### Embodiment 1:

During the production of vinyl acetate, oxygen is mixed with cycling gas containing ethylene and acetic acid through an oxygen mixer 4, mixed gas is introduced into a reactor 1 for reaction, the reacted high-temperature reaction gas is condensed through a condensing system 2, and uncondensed gas phase is recycled after being boosted by a cycling gas compressor 3. Nitrogen is introduced into the cycling gas to control the concentration of the nitrogen at the inlet of the reactor to be 30 % (mol%), and at that time the concentration of oxygen at the inlet of the reactor is improved to 12 % (mol%); at the initial period of using a catalyst, the reaction temperature is 110 °C; at the middle period of using the catalyst, the reaction temperature is 150 °C; and at the later period of using the catalyst, the reaction temperature is 180 °C. Compared with the traditional technology, the reaction temperature is reduced by 20 °C, the selectivity of the vinyl acetate is up to 96 % and improved by 3 %.

### Embodiment 2:

During the production of vinyl acetate, oxygen is mixed with cycling gas containing ethylene and acetic acid through an oxygen mixer 4, mixed gas is introduced into the reactor 1 for reaction, high-temperature reaction gas after reaction is condensed through a condensing system 2, and uncondensed gas phase is recycled after being boosted by a cycling gas compressor 3. Nitrogen is added into the cycling gas to control the concentration of the nitrogen at the inlet of the reactor to be 15 % (mol%), and at that time the concentration of oxygen at the inlet of the reactor is improved to 11.4 % (mol%); at the initial period of using a catalyst, the reaction temperature is 120 °C; at the middle period of using the catalyst, the reaction temperature is 160 °C; and at the later period of using the catalyst, the reaction temperature is 190 °C. Compared with the traditional technology, the reaction temperature is reduced by 10 °C, the selectivity of the vinyl acetate is up to 95 % and improved by 2 %.

### Embodiment 3:

During the production of vinyl acetate, oxygen is mixed with cycling gas containing ethylene and acetic acid through an oxygen mixer 4, mixed gas is introduced into the reactor 1 for reaction, high-temperature reaction gas after reaction is condensed through a condensing system 2, and uncondensed gas phase is recycled after being boosted by a cycling gas compressor 3. Nitrogen is added into the cycling gas to control the concentration of the nitrogen at the inlet of the reactor to be 8 % (mol%), and at that time the concentration of oxygen at the inlet of the reactor is improved to 10.5 % (mol%); at the initial period of using a catalyst, the reaction temperature is 125 °C; at the middle period of using the catalyst, the reaction temperature is 165 °C; and at the later period of using the catalyst, the reaction temperature is 195 °C. Compared with the traditional technology, the reaction temperature is reduced by 5 °C, the selectivity of the vinyl acetate is up to 93.5 % and improved by 0.5 %.

### Embodiment 4:

During the production of vinyl acetate, oxygen is mixed with cycling gas containing ethylene and acetic acid through an oxygen mixer 4, mixed gas is introduced into the reactor 1 for reaction, high-temperature reaction gas after reaction is condensed through a condensing system 2, and uncondensed gas phase is recycled after being boosted by a cycling gas compressor 3. A mixture of nitrogen and methane is added into the cycling gas to control the concentration of the mixture of nitrogen and methane at the inlet of the reactor to be 15 % (mol%), and at that time the concentration of oxygen at the inlet of the reactor is improved to 11 % (mol%); at the initial period of using a catalyst, the reaction temperature is 122 °C; at the middle period of using the catalyst, the reaction temperature is 162 °C; and at the later period of using the catalyst, the reaction temperature is 192 °C. The reaction temperature is reduced by 8 % compared with the traditional technology, the selectivity of the vinyl acetate is up to 94 % and improved by 1 %.

The method for producing vinyl acetate disclosed and provided by the present invention can be realized by using the content of the present invention for reference to properly change the links of raw materials, technological parameter and structural design by a person skilled in the art. The method and technology of the present invention have already been described through preferred embodiments, and a related person skilled in the art can obviously modify or properly change and combine the method and technology of the present invention without departing from the content, spirit and scope of the present invention so as to realize the technology of the present invention. Specifically, it is noted that all similar replacement and modification are obvious for the person skilled in the art and are all regarded to be included in the spirit, scope and content of the present invention.

## Claims

1. A method for producing vinyl acetate, wherein inert gas is introduced into cycling gas, and the concentration of the inert gas at the inlet of a reactor is 8 to 30 mol%.

2. The method for producing vinyl acetate of claim 1, wherein the inert gas is nitrogen or a mixture of nitrogen and methane.

3. The method for producing vinyl acetate of claim 2, wherein the volume ratio of nitrogen to methane is 4:1 to 4:2 when the introduced inert gas is the mixture of nitrogen and methane.

4. The method for producing vinyl acetate of claim 1, wherein the concentration of the oxygen at the inlet of the reactor is 10.5 to 12 mol%.

5. The method for producing vinyl acetate of claim 1, wherein the temperature of the reactor is 110-180 °C.
